(19) Europäisches Patentamt
European Patent Office
Office européen des brevets

(11) Veröffentlichungsnummer : **0 072 780**
**B1**

(12) **EUROPÄISCHE PATENTSCHRIFT**

(45) Veröffentlichungstag der Patentschrift : **30.01.85**

(51) Int. Cl.[4] : **C 07 D207/452**, C 07 D209/48, C 07 D209/52, C 08 G 65/26, C 08 G 65/28, C 08 F 26/06, C 08 F 8/32, G 03 C 1/71

(21) Anmeldenummer : **82810340.8**

(22) Anmeldetag : **11.08.82**

(54) **Imidylverbindungen, Polymere daraus und Verwendung der Polymeren.**

(30) Priorität : **17.08.81 CH 5303/81**

(43) Veröffentlichungstag der Anmeldung : **23.02.83 Patentblatt 83/08**

(45) Bekanntmachung des Hinweises auf die Patenterteilung : **30.01.85 Patentblatt 85/05**

(84) Benannte Vertragsstaaten : **BE CH DE FR GB IT LI NL SE**

(56) Entgegenhaltungen :
**EP-A- 0 002 439**
**DE-A- 2 349 948**
**DE-A- 2 626 769**
**DE-A- 2 626 795**
**DE-A- 2 834 919**
**DE-A- 3 008 657**
**DE-B- 2 437 368**
**DE-B- 2 437 422**
**US-A- 3 520 852**
**US-A- 3 972 961**

(73) Patentinhaber : **CIBA-GEIGY AG**
**Postfach**
**CH-4002 Basel (CH)**

(72) Erfinder : **Müller, Beat, Dr.**
**Chemin des Cassettes 7**
**CH-1723 Marly (CH)**

**EP 0 072 780 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die vorliegende Erfindung betrifft Imidylverbindungen mit Hydroxyl- oder Aethenylgruppen als funktionellen Gruppen, Homo- oder Copolymeren daraus und deren Verwendung als photographisches Aufzeichnungsmaterial.

Die Bedeutung von lichtempfindlichen Polymeren hat in der Technik insbesondere zur Herstellung von z. B. gedruckten Schaltungen und Druckplatten ständig zugenommen. Solche Photopolymeren sollen wirtschaftlich verarbeitet werden können. Die Entwickelbarkeit der durch Belichtung vernetzten Polymeren wird hauptsächlich durch die Schichtdicke, die Entwicklungszeit und -temperatur bestimmt, die für eine wirtschaftliche Verarbeitung nicht zu lange bzw. zu hoch sein dürfen. Die mechanische Beanspruchung während der Verarbeitung erfordert auch eine gute Haftfestigkeit auf dem Substrat, besonders bei höheren Schichtdicken. Für die Verarbeitung ist es ferner günstig, wenn bezüglich der Entwicklung ein gewisser Spielraum vorhanden ist und somit das gewünschte Ergebnis nicht von der genauen Einhaltung kritischer Grenzwerte abhängig ist.

In den deutschen Offenlegungsschriften 26 26 795 und 26 26 769 sind Imidylverbindungen und Copolymere daraus beschrieben, die als photographisches Aufzeichnungsmaterial verwendet werden können. Unter anderen sind auch N-(Hydroxyäthyl)dimethylmaleinimid und N-(β-Hydroxypropyl)dimethylmaleinimid, deren Acrylsäure- und Methacrylsäureester und Copolymere davon beschrieben. Eigene Untersuchungen haben ergeben, dass nur innerhalb enger Grenzen der Entwicklungsbedingungen eine ausreichende Bildqualität erhalten und ausserhalb dieser Grenzen entweder die Bildung von Schleiern oder eine Quellung des verntzten Polymeren beobachtet wird. Auch die Haftfestigkeit der lichtvernetzbaren Polymerschicht auf dem Substrat ist besonders bei höheren Schichtdicken nicht ausreichend.

Aufgabe vorliegender Erfindung ist es, Imidylverbindungen für lichtvernetzbare Polymere bereitzustellen, die als photographisches Aufzeichnungsmaterial innerhalb erweiterter Entwicklungsgrenzen auch bei höheren Schichtdicken keine Schleierbildung und Quellung zeigen und die besser auf dem Substrat haften.

Gegenstand vorliegender Erfindung sind Imidylverbindungen oder Mischungen von Imidylverbindungen der Formel I

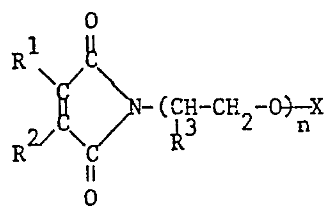

(I),

worin

$R^1$ und $R^2$ unabhängig voneinander $C_1$-$C_4$-Alkyl oder

$R^1$ und $R^2$ zusammen unsubstituiertes oder durch $C_1$-$C_4$-Alkyl substituiertes Tri- oder Tetramethylen bedeuten,

$R^3$ ein Wasserstoffatom oder $C_1$-$C_4$-Alkyl ist,

n eine Zahl von 2 bis 30 ist und

X ein Wasserstoffatom oder $CO—CR^4=CH_2$ bedeutet, wobei $R^4$ ein Wasserstoffatom oder Methyl ist.

Unter den Mischungen der Imidylverbindungen sind jene bevorzugt, in denen n verschieden Zahlen von 2 bis 30 bedeutet. Bevorzugt bedeutet n eine Zahl von 2 bis 20, besonders 2 bis 10 insbesondere 2 bis 6.

$R^1$ und $R^2$ können als Alkyl Methyl, Aethyl, Propyl, i-Propyl, n-Butyl, i-Butyl und t-Butyl sein. Die gleiche Bedeutung kann der Alkylsubstituent haben, wenn $R^1$ und $R^2$ zusammen Tri- oder Tetramethylen sind. Bevorzugt ist das Trimethylen und Tetramethylen monoalkyliert und bevorzugt ist der Alkylsubstituent Methyl. $R^1$ und $R^2$ sind bevorzugt Methyl und zusammen unsubstituiertes oder methyliertes 1,4-Butylen.

$R^3$ in seiner Bedeutung als Alkyl kann z. B. Methyl, Aethyl, n-Propyl oder n-Butyl sein. Bevorzugt ist $R^3$ Methyl und insbesondere ein Wasserstoffatom.

Eine bevorzugte Gruppe von Verbindungen der Formel I sind solche der Formel

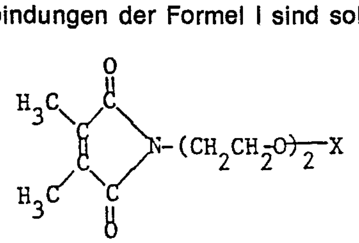

,

worin X die zuvor angegebene Bedeutung hat.

Die Verbindungen der Formel I können nach analogen Verfahren gemäss DE-A 26 26 795 hergestellt

werden, indem man mindestens stöchiometrische Mengen Poly(oxyalkylen)amin der Formel II

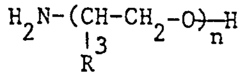

(II)

mit einem Dicarbonsäureanhydrid der Formel III

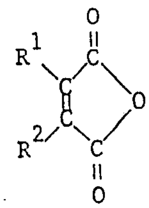

(III)

umsetzt. Die Umsetzung erfolgt vorteilhaft bei Temperaturen von 50° bis 200 °C, vorzugsweise 70° bis 150 °C, in Gegenwart eines inerten Lösungsmittels mittels destillativer Entfernung des entstehenden Reaktionswassers. In einer anderen Ausführungsform zur Herstellung der Verbindungen der Formel I, worin X = H ist, kann man so vorgehen, dass man an Imidylverbindungen, worin X in Formel I H und n die Zahl 1 oder eine Zahl > 1 bedeutet, gegebenenfalls $C_1$-$C_4$-alkyliertes Aethylenoxid anlagert. Zur Herstellung der Verbindungen der Formel I, worin X Methacryloyl oder Acryloyl ist, können die Alkohole der Formel I mit (Meth)Acrylsäure oder deren esterbildenden Derivaten wie z. B. Säurehalogenide, -ester und anhydride, verestert werden.

Geeignete Lösungsmittel sind z. B. Aether wie Diäthyläther, Dibutyläther, Tetrahydrofuran und Dioxan und Kohlenwasserstoffe wie Pentan, Cyclohexan, Benzol, Toluol und Xylol.

Die Poly(oxyalkylen)amine der Formel II sind bekannt oder können nach an sich bekannten Verfahren durch Umsetzung von gegebenenfalls $C_1$-$C_4$-alkylierten N-geschützten Aminoäthanolen mit (n − 1) Molen eines gegebenenfalls $C_1$-$C_4$-alkylierten Aethylenoxids erhalten werden.

Die Anhydride der Formel III sind bekannt.

Die Verbindungen der Formel I sind je nach der Höhe des Zahlenwertes für n von flüssiger, viskoser bis harzartiger Konsistenz. Sie eignen sich besonders zur Herstellung von lichtvernetzbaren Homo- und Copolymeren, die ausgezeichnete photographische Aufzeichnungsmaterialien sind.

Ein weiterer Gegenstand vorliegender Erfindung sind lichtempfindliche vernetzbare Homopolymere aus Verbindungen der Formel I und Copolymere mit äthylenisch ungesättigten Comonomeren. Die Polymeren können ein mittleres Molekulargewicht von 1 000 bis 1 000 000, vorzugsweise 5 000 bis 500 000 aufweisen.

Die erfindungsgemässen Copolymeren können je nach Verwendungszweck die lichtempfindlichen Verbindungen der Formel I in einer Menge von 1 bis 99 Gew.% , vorzugsweise 5 bis 95 Gew.% und besonders 20 bis 90 Gew.% und die Comonomeren in einer Menge von 99 bis 1 Gew.%, vorzugsweise 95 bis 5 Gew.% und besonders 80 bis 10 Gew.% enthalten, bezogen auf das Polymer. Das gewünschte Eigenschaftsbild des Copolymeren kann erhalten werden, indem man ein oder mehrere Verbindungen der Formel I und Comonomere zur Herstellung der Copolymeren verwendet.

Geeignete Comonomere sind z. B. α-Olefine wie Aethylen, Propylen, n-Butylen, Isobutylen, Pentylen und Hexylen, Vinylhalogenide wie Vinylchlorid, Vinylbromid, Vinylfluorid, Tetrafluoräthylen und Vinylidenchlorid, aromatische Vinylverbindungen wie Styrol, Methylstyrol, Vinyltoluol oder α-Chlorstyrol, heterocyclische Vinylverbindungen wie Vinylpyrrolidon, Vinylcarbazol, Vinylpyridin, Vinylimidazol, Vinylketone wie Methylvinylketon, Vinylester wie Vinylacetat, Vinyläther wie Vinylmethyläther, Vinylsulfonsäuren, Allylverbindungen und Vinylglycidyläther sowie gegebenenfalls Diene wie Butadien, Chlorbutadien, Isopren oder Chloropren.

Eine bevorzugte Gruppe von Vinylmonomeren sind die α,β-ungesättigten Carbonsäuren und deren Derivate, z. B. Acrylsäure, Methacrylsäure, Crotonsäure, Maleinsäure oder Fumarsäure, Dicarbonsäureanhydride wie Maleinsäureanhydrid, α,β-ungesättigte Nitrile wie Acrylnitril, Methacrylnitril, Crotonnitril, α,β-ungesättigte Carbonsäureamide wie Acrylsäureamid, Methacrylsäureamid, Crotonsäureamid und α,β-ungesättigte Carbonsäureester wie Methylacrylat, Aethylacrylat, Propylacrylat, Butylacrylat, Pentylacrylat, Hexylacrylat, Octylacrylat, 2-Aethylhexylacrylat, Isopropylacrylat, Isobutylacrylat und die analogen Methacrylsäure- und Crotonsäureester.

Eine besonders bevorzugte Untergruppe sind Copolymere aus Verbindungen der Formel I mit Acrylsäure, Methacrylsäure, Acrylsäureester und/oder Methacrylsäureester. Die Verbindungen der Formel I können vorzugsweise in einer Menge von 40 bis 90, besonders 60 bis 90 Gew.%, die Acryl- und Methacrylsäure in einer Menge von 5 bis 20, vorzugsweise 5 bis 30 Gew.% und die Acryl- und Methacrylsäureester in einer Menge von 5 bis 20, vorzugsweise 5-30 Gew.%, enthalten sein, bezogen auf das Polymer.

Die erfindungsgemässen Polymeren lassen sich nach an sich bekannten Synthesemethoden zur Herstellung von Makromolekülen mit photoaktiven seitenständigen Gruppen herstellen. Solche Verfahren sind z. B. in der DE-A 26 26 769 beschrieben. Grundsätzlich kommen folgende Wege in Betracht :

1. Einbau von Verbindungen der Formel I, in denen X Wasserstoff bedeutet, in eine bestehende Polymerkette.

2. Aufbau der Polymerkette durch Unipolymerisation von Verbindungen der Formel I, in denen X (Meth)Acryloyl ist, oder Copolymerisation mit Comonomeren.

Mit beiden Ausführungsformen können zum Teil gleiche Produkte erhalten werden. Die Hydroxygruppe in den Verbindungen der Formel I kann zur Verwendung in der ersten Ausführungsform auch in geeignete Derivate mit anderen reaktiven Gruppen umgewandelt werden, z. B. in Dicarbonsäurehalbester, —COCl und Glycidyläther.

Werden die lichtempfindlichen Verbindungen der Formel I bzw. geeignete Derivate durch eine Sekundärreaktion in eine bereits vorhandene Polymerkette eingebaut, so erfolgt dieser Einbau entweder durch eine Kondensationsreaktion unter Austritt von $H_2O$, Alkoxy, Halogenwasserstoff oder dgl., oder durch eine Additionsreaktion unter gleichzeitiger Oeffnung eines Ringsystems, wie z. B. einer Dicarbonsäureanhydridgruppe oder einer Epoxidgruppe.

Als für die Einführung der lichtempfindlichen Verbindungen der Formel I durch Kondensation oder Addition geeignete Ausgangspolymere sind folgende Produkte aufzuzählen: Polyacrylsäure, Polymethacrylsäure und deren Ester, Copolymere aus diesen Säuren und anderen äthylenisch ungesättigten Monomeren, Copolymere, aufgebaut aus Maleisäureanhydrid und äthylenisch ungesättigten Monomeren wie Methylvinyläther, Aethylen, Styrol, Hexen-1, Decen-1, Tetracen-1 und Octadecen-1, mit freien Hydroxylgruppen wie Uni- oder Copolymere von Acrylsäure- und Methacrylsäurehydroxyalkylestern, Polyvinylalkohole, Polymere mit freien Glycidylgruppen wie Copolymere auf Basis von Acryl- und Methacrylsäureglycidylestern und Polyvinylglycidyläther.

Die Polymerisation gemäss der zweiten Ausführungsform kann mittels radikalischer Katalyse, bevorzugt in Lösung durchgeführt werden, ohne dass hierbei vorzeitige Vernetzungsreaktionen beobachtet werden. Die Reaktionsbedingungen, z. B. Mengenverhältnisse, Temperatur, Katalysatoren, Lösungsmittel und dgl. sind ausführlich in der DE-A 26 26 769 beschrieben. Die Polymerisate können durch übliche Aufarbeitungsmethoden isoliert werden, wie z. B. Fällung aus der Lösung oder destillative Entfernung des Lösungsmittels.

Die erfindungsgemässen Polymeren eignen sich für verschiedene Verwendungen. Durch die vorhandenen lichtempfindlichen Maleinimidgruppen eignen sie sich besonders zur Vernetzung unter dem Einfluss elektromagnetischer Wellen. Die Vernetzung führt zu unlöslichen Produkten. Durch bildmässige Belichtung und nachfolgende Entwicklung (Herauslösen des unbelichteten, unvernetzten Polymeranteils) können so Reliefbilder erhalten werden. Neben der Verwendung als photographisches Aufzeichnungsmaterial können die erfindungsgemässen Polymeren auch als Klebmittel und zum Oberflächenschutz verschiedener Substrate wie Kunststoffe, Metalle, Glas, Holz und Keramik eingesetzt werden. Beim Einsatz als Klebmittel muss mindestens ein Substrat transparent sein.

Den Polymeren können übliche Zusatzmittel einverleibt werden, die die Lichtempfindlichkeit nicht negativ beeinflussen. Beispiele hierfür sind insbesondere Sensibilisatoren, wie z. B. in der DE-A 26 26 769 beschrieben sind, sowie Mattierungsmittel, Verlaufmittel, Füllstoffe, Flammschutzmittel, optische Aufheller, Antioxidantien, Lichtschutzmittel und Verarbeitungsstabilisatoren.

Die lichtempfindliche Schicht kann mittels üblichen Methoden wie Tauch- und Sprühverfahren, Schleuder-, kaskadenguss- und Vorhanggussbeschichtung auf geeignete Substrate bzw. Trägermaterialien aufgebracht werden.

Die Anwendungsmöglichkeiten der erfindungsgemässen Polymeren liegen z. B. auf dem Gebiet der Photofabrikation, der Druckplattenherstellung und der Nichtsilberphotographie. Bei der Nichtsilberphotographie kann nach dem Belichten und Entwickeln das kaum bis schlecht sichtbare Polymerbild durch Anfärben mit öllöslichen Farbstoffen oder, wenn das Polymer saure Gruppen wie Carbonsäuregruppen enthält, durch Anfärben mit kationischen Farbstoffen gut sichtbar gemacht werden.

Das photographische Aufzeichnungsmaterial eignet sich besonders zur Herstellung von Photomasken für die Elektronik, den Textildruck und das graphische Gewerbe.

Die erfindungsgemässen Polymeren zeichnen sich durch eine hohe Haftfestigkeit aus. Als photographisches Aufzeichnungsmaterial liefern sie auch bei höheren Schichtdicken sehr gute Bildqualitäten. Bei höheren Badtemperaturen wird schon bei kurzen Verweilzeiten eine vollständige Bildentwicklung und selbst bei längeren Verweilzeiten keine Quellung der belichteten Polymeranteile beobachtet. Mit fallender Temperatur ist eine längere Verweilzeit zur Entwicklung erforderlich. Besonders vorteilhaft ist, dass die Entwicklungsbedingungen so gewählt werden können, dass Prozessschwankungen keinen negativen Einfluss auf die gute Bildqualität haben.

Die nachfolgenden Beispiele erläutern die Erfindung näher.

## Beispiel 1

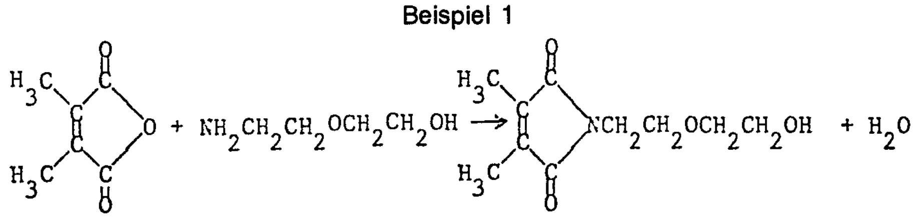

63,05 g (0,5 Mol) Dimethylmaleinsäureanhydrid werden in 250 ml Toluol in einem Dreihalskolben mit Rührer, Thermometer und Wasserabscheider vorgelegt. Dazu werden 53,61 g (0,51 Mol) Diglycolamin während 10 Min. zugegeben. Die Mischung wird anschliessend während 1 Stunde am Rückfluss gekocht, dabei scheiden sich ca. 9 ml Wasser ab. Um das überschüssige Diglycolamin zu entfernen, werden 5 g eines sauren Ionenaustauscherharzes zugegeben und 10 Min. gerührt ; dann wird die Mischung abgekühlt und das Ionenaustauscherharz abfiltriert. Das Produkt ist zu mehr als 98 % rein (gaschromatographische Bestimmung). Nach Enfternung des Toluols wird das Produkt destilliert. Siedepunkt : 145 °C/66,66 Pa. Ausbeute 95 %. Das NMR-Spektrum stimmt mit der Struktur überein.

Analyse :

| | ber. | gef. |
|---|---|---|
| % C | 56,33 | 56,11 |
| % H | 7,09 | 7,23 |
| % N | 6,57 | 6,66 |

Beispiel 2

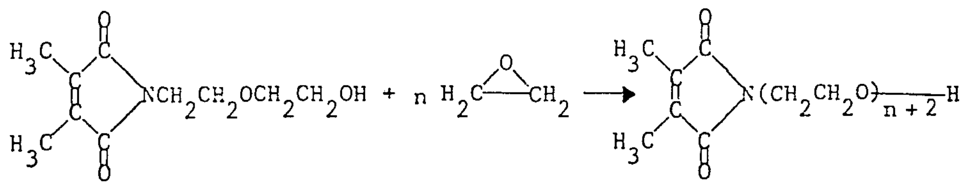

Zu 263 g des Dimethylmaleinsäureimidylalkohols gemäss Beispiel 1 werden 2,6 ml frisch destilliertes Bortrifluorid-Aetherat zugegeben und bei 70-80° unter starkem rühren 101 g Aethylenoxid währen 1 1/2 Stunden eingeleitet. Das Reaktionsgemisch wird fraktionniert destilliert. Es können fünf verschiedene Fraktionen abgetrennt werden (n = 2, 3, 4, 5, 6).

| Siedepunkt | Druck (in Pa) | Produkt mit n gleich | Menge | |
|---|---|---|---|---|
| 125-126 °C | 2,66 | 2 | 80 g | |
| 150-153 °C | 0,66 | 3 | 74 g | |
| 191-192 °C | 0,53 | 4 | 100 g | |
| 212 °C | 0,39 | 5 | 54 g | |
| 250 °C [1)] | 6,66 | 6 | 25 g | |
| Rückstand | | | 27 g | |
| | | | 360 g | ≙ 98 % d. Th. |

[1)] Beginnende Zersetzung.

Analyse :

| | | ber. | gef. |
|---|---|---|---|
| n = 3 | % C | 56,02 | 55,86 |
| | % H | 7,44 | 7,44 |
| | % N | 5,44 | 5,76 |
| n = 4 | % C | 55,80 | 56,64 |
| | % H | 7,69 | 7,69 |
| | % N | 4,65 | 4,71 |
| n = 5 | % C | 55,64 | 55,49 |
| | % H | 7,88 | 7,88 |
| | % N | 4,05 | 4,10 |

Die NMR-Spektren bestätigen die Strukturen.

Beispiel 3

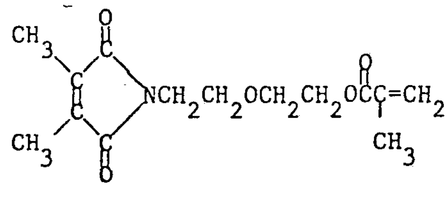

In einem Dreihalskolben mit mechanischem Rührer, Wasserabscheider und Thermometer wird 106,6 g (0,5 Mol) des Dimethylmaleinimidylalkohols aus Beispiel 1 in 250 ml Toluol vorgelegt und mit 47,34 g (0,55 Mol) Methacrylsäure, 120 mg Di-tert.-butyl-p-Kresol und 3 ml Schwefelsäure versetzt. Die Mischung wird während zwei Studen am Rückfluss gekocht. Es scheidet sich ca. 8,8 ml Wasser ab. Nach Abkühlen im Eisbad werden 100 ml Petroläther zugegeben und die Mischung wird zweimal mit Wasser, einmal mit Natronlauge (1 N) und nochmals zweimal mit Wasser ausgerührt. Die farblose Toluollösung wird am Rotationsverdampfer eingedampft. Es resultiert nach Entfernen der Lösungsmittelreste am Hochvakuum 116 g polymerfreies Rohprodukt (83 % Ausbeute, 98 % Reinheit), welches für die Polymerisation ohne Destillation verwendet werden kann.

Das Produkt destilliert bei 140-142 °C 6,66 Pa (0,05 mm/Hg)

Analyse :

| | ber. | gef. |
|---|---|---|
| % C | 59,78 | 59,68 |
| % H | 6,81 | 6,64 |
| % N | 4,98 | 4,94 |

Das NMR-Spektrum stimmt mit der Struktur überein.

Die in Tabelle 1 angegebenen höheren homologen Verbindungen mit n = 3, 4 und 5 werden nach dem gleichen Verfahren aus den entsprechenden Alkoholen des Beispiels 2 hergestellt.

Tabelle 1

| 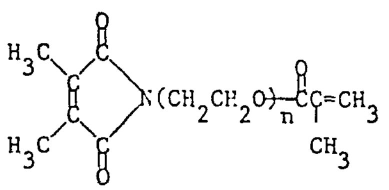 | Ausbeute | NMR-Signal bei 3,6 u. 4,3 ppm entsprechen $-O-CH_2CH_2O-$ / Anzahl der Protonen | | |
|---|---|---|---|---|
| | | 3,6 ppm | | 4,3ppm |
| n = 3 | 80 % | 10 | Protonen | 2 |
| 4 | 76 % | 14 | Protonen | 2 |
| 5 | 72 % | 18 | Protonen | 2 |

Beispiel 4

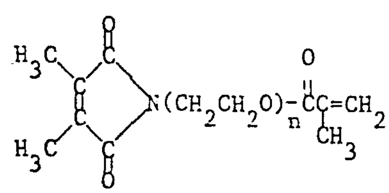

(Gemische homologer Verbindungen mit n = 2, 3, 4 etc.)

In einem Dreihalskolben mit Rührer, Wasserabscheider und Thermometer werden 106 g (0,5 Mol) Dimethylmaleinimidylalkohol gemäss Beispiel 1 vorgelegt und in 250 ml Toluol gelöst. Es werden 2 ml Bortrifluorid-Aetherat zugegeben und 22 g (0,5 Mol) Aethylenoxid unter kräftigem Rühren bei 70 °C während 20 Minuten eingeleitet. Das so erhaltene Alkoholgemisch wird analog Beispiel 3 mit Methacrylsäure verestert. Nach Isolation resultieren 135 g polymerfreies Produkt (80 % Ausbeute), welches direkt für die Polymerisation verwendet werden kann. Nach Dünnschichtchromatogramm und Gaschromatogramm handelt es sich um ein Gemisch von Verbindungen mit hauptlächlich n = 2, 3, 4, 5 und 6.

Durch Variation der zugegebenen Aethylenoxidmenge können auf analoge Art verschiedene Homologengemische hergestellt werden, die sich in den Hydrophilie unterscheiden.

## Polymerisationsbeispiele

### Beispiel 5

In einem 500 ml Doppelmantelreaktor mit Heizung, Rührer, Thermometer, Tropftrichter, Kühler und $N_2$-Anschluss werden vorgelegt :

48,75 g Dimethylimidylmethacrylat aus Beispiel 3 (nicht destilliert) (65 %)
15 g Methylmethacrylat (20 %)
11,25 g Methacrylsäure (15 %)
291 g einer 1 : 1-Mischung (Volumenverhältnis) von Methylcelosolve (MCS) und Methyläthylketon (MEK)

Im Tropftrichter wird eine Lösung von 450 mg α,α'-Azo-isobutyronitril (AIBN) in 9 g des gleichen Lösungsmittelgemisches vorgelegt.

Die Apparatur wird dreimal evakuiert und mit Stickstoff gespült und dann unter Stickstoffüberdruck gehalten.

Das Reaktionsgemisch wird auf 65 °C erwärmt und 1/3 der AIBN-Lösung zugegeben. Nach fünf und nach zehn Stunden wird noch je ein Drittel der AIBN-Lösung zugegeben. Nach 22 Std. Reaktionszeit wird die Polymerlösung abgekühlt.

Die viskose, farblose Polymerlösung wird mit Aether versetzt und das gefällte feste Polymer abfiltriert und getrocknet.

Die Polymerausbeute ist ca. 95 %.

Die inhärente Viskosität ist 0,263 dl/g (0,5 % in MCS bei 25 °C). Der Säuregehalt wird durch Titration bestimmt und ist 14,9 %.

In Tabelle 2 sind weitere Polymere aufgeführt, welche analog der obigen Vorschrift hergestellt werden.

Tabelle 2

| Nr. | Eingesetzte Monomermischung (Gew.%) | Konzentration der Polymerisationslösung in Gew.% | Inhärente Viskosität | Säuregehalt % | Ausbeute % |
|---|---|---|---|---|---|
| 1 | Monomeres aus Beispiel 3 75%<br>Methylmethacrylat 10%<br>Methacrylsäure 15% | 15% | 0.18 | 13.9 | 94 |
| 2 | Monomeres aus Beispiel 3 65%<br>Methylmethacrylat 20%<br>Methycrylsäure 15% | 15% | 0.19 | 14.6 | 95 |
| 3 | Monomeres aus Beispiel 3 90%<br>Methacrylsäure 10% | 15% | 0.18 | - | 94 |
| 4 | Monomeres aus Beispiel 3 50%<br>Methylmethacrylat 30%<br>Methacrylsäure 20% | 15% | 0.25 | 18.9 | 90 |

Tabelle 2 (Fortsetzung)

| Nr. | Eingesetzte Monomermischung (Gew.%) | Konzentration der Polymerisationslösung in Gew.% | Inhärente Viskosität | Säuregehalt % | Ausbeute % |
|---|---|---|---|---|---|
| 5 | Monomeres aus Beispiel 3 85%<br>Methacrylsäure 15% | 15% | 0.18 | - | 96 |
| 6 | Monomeres aus Beispiel 3 80%<br>Methacrylsäure 20% | 15% | 0.22 | - | 95 |
| 7 | Monomeres aus Beispiel 3 60%<br>Aethylacrylat 20%<br>Methacrylsäure 20% | 15% | 0.22 | - | 86 |
| 8 | Monomeres aus Beispiel 3 65%<br>Methylmethacrylat 20%<br>Methacrylsäure 15% | 25% | 0.30 | 14.2 | 94 |
| 9 | Monomeres aus Beispiel 3 65%<br>Methylmethacrylat 20%<br>Methacrylsäure 15% | 30% | 0.40 | 14.8 | 95 |
| 10 | Monomeres aus Beispiel 3 90%<br>Methacrylsäure 10% | 30% | 0.33 | - | 95 |
| 11 | Monomergemisch aus Beispiel 4 (1.5 Aequivalente Aethylenoxid) 65%<br>Methylmethacrylat 20%<br>Methacrylsäure 15% | 20% | 0.35 | 16 | 90% |
| 12 | Monomergemisch aus Beispiel 4 (1.0 Aequivalente Aethylenoxid) 65%<br>Methylmethacrylat 20% | 20% | 0.27 | 16.7 | - |

Tabelle 2 (Fortsetzung)

| Nr. | Eingesetzte Monomermischung (Gew.%) | Konzentration der Polymerisationslösung in Gew.% | Inhärente Viskosität | Säuregehalt % | Ausbeute % |
|---|---|---|---|---|---|
| | Methacrylsäure 15% | | | | |
| 13 | Monomergemisch aus Beispiel 4 (1.0 Aequivalente Aethylenoxid) 95%<br>Methacrylsäure 5% | 20% | 0.24 | 5.4 | 18% |
| 14 | Monomeres aus Beispiel 3 70%<br>4-Vinyl-pyridin 30% | 20% | 0.18 | - | 70% löslich in 1NHCl |
| 15<br>15 | Monomeres aus Beispiel 3 50%<br>1-Vinyl-2-pyrrolidon 50% | 20% | 0.12 | - | 55% |
| 16 | Momomeres aus Beispiel 3 70%<br>1 Vinyl-2-pyrrolidon 30% | 20% | 0.14 | - | 80% |

Anwendungsbeispiel

Beispiel 6

Die Polymerlösung, wie in Beispiel 5 hergestellt, wird mit dem Lösungsmittelgemisch Methylcelosolve/Methyläthylketon 1 : 1 auf 7 % Feststoffgehalt verdünnt. Bei Gelblicht wird 8 % des Thioxantonsensibilisators bezogen auf den Feststoffgehalt,

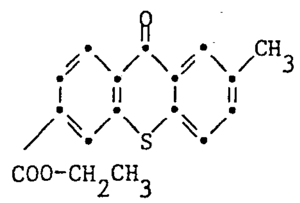

zugegeben sowie handelsübliche Mattierungs- und Verlaufmittel.

Die Polymerlösung wird dann mit einer Beschichtungsmaschine auf eine 100 μ dicke Polyesterfolie aufgetragen und bei 110 °C getrocknet. Die Schichtdicke entspricht einem Polymerauftrag von 3 g/m². Der so hergestellte Film wird durch eine Bildmaske 20 sec. mit einer 1 000 W Metallhalogenlampe im Abstand von 80 cm belichtet. Danach wird bei 40 °C in einer 5 %-igen Natriumcarbonatlösung entwickelt und das erhaltene Polymer-Relief wird bei Raumtemperatur mit Wasser gewaschen. Mit einer wässrigen Lösung aus handelsüblichen Maxilonfarbstoffen wird das Polymer-Relief angefärbt, nach Abwaschen des überschüssigen Farbstoffs wird der Film getrocknet.

Die optische Dichte des so behandelten Films ist im Wellenlängenbereich von 350-500 nm ≥ 3.

Beispiel 7

Es werden mit der in Beispiel 6 verwendeten Polymerlösung Filme verschiedener Polymerauftragsmengen hergestellt : 2,8 ; 3,0 ; 3,3 ; 3,9 g/$m^2$. Die Filme werden nach bildmässiger Belichtung mit einer 5 %-igen Natriumcarbonat-Lösung bei verschiedenen Verweilzeiten und verschiedenen Badtemperaturen entwickelt. Das Ergebnis ist in der nachfolgenden Tabelle 3 dargestellt.

Tabelle 3

| Bad-temepratur | Verweilzeit | | | |
|---|---|---|---|---|
| | 15 sec. | 18 sec. | 25 sec. | 38 sec. |
| 40°C | gut | gut | gut | gut |
| 35°C | Schleier | gut | gut | gut |
| 30°C | Schleier | Schleier | Schleier | gut |

Das Polymere ist bei verschiedenen Schichtdicken in einem grossen Temperaturbereich und Verweilzeitspielraum entwickelbar.

Das analoge Polymere, mit vergleichbarer Viskosität und Säuregehalt, jedoch dem nächst niedrigen homologen Monomeren Beispiel 4, (n = 1) ist in Natriumcarbonat nicht entwickelbar. Wird der Säuregehalt auf 20 % erhöht, damit es unter den angewendeten Bedingungen löslich wird, so ist der Verweilzeitspielraum sehr klein ($\leq$ 5 sec.).

Der Vorteil der beschriebenen Polymeren liegt in der guten Haftung der Polymerschicht bei wässrigalkalischer Entwicklung und in der guten and raschen Löslichkeit des unbelichteten Polymeren. Die Verbesserung der Haftung zeigt sich besonders bei dickeren Schichten.

Beispiel 8

Die in Beispiel 5, Tabelle 2, Nr. 2 hergestellte Polymerlösung wird mit 5 % Sensibilisator (Analog Beispiel 6) sensibilisiert und mit 0,25 % Ovasolrot B und 0,5 % Di-tert.-butyl-p-kresol versetzt.

Mit der so erhaltenen Lösung wird eine Aluminiumplatte beschichtet und anschliessend bei 80 °C während 3 Minuten getrocknet. Die aufgebrachte Polymermenge entspricht ca. 3 g/$m^2$. Die so hergestellte Druckplatte wird mit einer 1 000 W-Metallhalogenlampe in 80 cm Abstand während 20 sec. durch eine Vorlage hindurch belichtet, hernach in einer 1 %-igen Natriumcarbonat-Lösung 1-2 Minuten entwickelt und mit Wasser gewaschen. Nach kurzem Eintauchen in 1 %-ige Phosphorsäure wird das Polymerrelief mit einer Druckfarbe eingefärbt.

Beispiel 9

Zur Polymerlösung aus Beispiel 5, Tabelle 2, Nr. 11 wird 2 % Sensibilisator (siehe Beispiel 6) und 0,2 % Ovasolrot B zugegeben. Eine Leiterplatte wird mit dieser Polymerlösung beschichtet, und anschliessend bei 85 °C 25 Minuten getrocknet. Die aufgebrachte Schichtdicke entspricht einem Polymerauftrag von 32 g/$m^2$. Die Leiterplatte wird mit einer 1 000 W-Metallhalogenidlampe 100 Sekunden in 80 cm Abstand bildmässig belichtet und anschliessend bei 30 °C in einer 1 %-igen Natriumcarbonatlösung während 1,5 Minuten entwicklet. Es entsteht ein gut haftendes Polymerrelief, auf das in einem Galvanisierbad Kupfer abgeschieden werden kann.

**Ansprüche**

1. Imidylverbindungen oder Mischung von Imidylverbindungen der Formel I

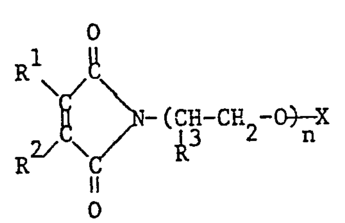

(I)

worin

$R^1$ und $R^2$ unabhängig voneinander $C_1$-$C_4$-Alkyl oder $R^1$ und $R^2$ zusammen unsubstituiertes oder durch $C_1$-$C_4$-Alkyl substituiertes Tri- oder Tetramethylen bedeuten,

$R^3$ ein Wasserstoffatom oder $C_1$-$C_4$-Alkyl ist,
n eine Zahl von 2 bis 30 ist und
X ein Wasserstoffatom oder $CO—CR^4{=}CH_2$ bedeutet, wobei $R^4$ ein Wasserstoffatom oder Methyl ist.

2. Verbindungen gemäss Anspruch 1, dadurch gekennzeichnet, dass $R^1$ und $R^2$ Methyl oder zusammen unsubstituiertes oder methyliertes 1,4-Butylen bedeuten.

3. Verbindungen gemäss Anspruch 1, dadurch gekennzeichnet, dass $R^3$ Methyl und insbesondere ein Wasserstoffatom ist.

4. Verbindungen gemäss Anspruch 1, worin n eine Zahl von 2 bis 20, besonders 2 bis 10 und insbesondere 2 bis 6 ist.

5. Verbindungen gemäss Anspruch 1 der Formel

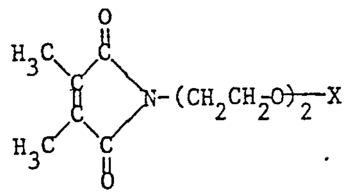

,

worin X die in Anspruch 1 angegebene Bedeutung hat.

6. Lichtempfindliche vernetzbare Polymere aus Verbindungen der Formel I gemäss Anspruch 1 und Copolymere mit äthylenisch ungesättigten Comonomeren.

7. Polymere gemäss Anspruch 6, dadurch gekennzeichnet, dass sie ein Molekulargewicht von 1 000 bis 1 000 000 aufweisen.

8. Polymere gemäss Anspruch 6, dadurch gekennzeichnet, dass das ungesättigte Comonomer im Copolymer ein Vinylmonomer ist.

9. Polymere gemäss Anspruch 8, dadurch gekennzeichnet, dass das Vinylmonomer Acrylsäure oder Methacrylsäure und/oder ein Acrylsäureester oder Methacrylsäureester ist.

10. Polymere gemäss Anspruch 9, dadurch gekennzeichnet, dass die Verbindung der Formel I in einer Menge von 40 bis 90, vorzugsweise 60 bis 90 Gew.%, die Acryl- oder Methacrylsäure in einer Menge von 5 bis 20, vorzugsweise 5 bis 30 Gew.% und der Acryl- bzw. Methacrylsäureester in einer Menge 5 bis 20, vorzugsweise 5 bis 30 Gew.% enthalten ist, bezogen auf das Copolymer.

11. Verwendung von Polymeren gemäss Anspruch 6 als photographisches Aufzeichnungsmaterial.

**Claims**

1. An imidyl compound or a mixture of imidyl compounds of the formula I

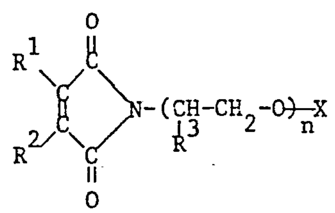

(I)

wherein
$R^1$ and $R^2$ independently of one another are $C_1$-$C_4$-alkyl, or
$R^1$ and $R^2$ together are tri- or tetramethylene which is unsubstituted or substituted by $C_1$-$C_4$-alkyl,
$R^3$ is a hydrogen atom or $C_1$-$C_4$-alkyl,
n is a number from 2 to 30, and
X is a hydrogen atom or $CO—CR^4{=}CH_2$, in which $R^4$ is a hydrogen atom or methyl.

2. A compound according to Claim 1, wherein $R^1$ and $R^2$ are methyl, or together are unsubstituted or methylated 1,4-butylene.

3. A compound according to Claim 1, wherein $R^3$ is methyl and in particular a hydrogen atom.

4. A compound according to Claim 1, wherein n is a number from 2 to 20, especially 2 to 10, and in particular 2 to 6.

5. A compound according to Claim 1 of the formula

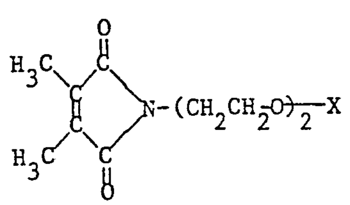

,

wherein X has the meaning defined in Claim 1.

6. A photosensitive, crosslinkable polymer formed from a compound of the formula I according to Claim 1, and a copolymer thereof with an ethylenically unsaturated comonomer.

7. A polymer according to Claim 6, which polymer has a molecular weight of 1,000 to 1,000,000.

8. A polymer according to Claim 6, wherein the unsaturated comonomer in the copolymer is a vinyl monomer.

9. A polymer according to Claim 8, wherein the vinyl monomer is acrylic acid or methacrylic acid and/or an acrylic acid ester or methacrylic acid ester.

10. A polymer according to Claim 9, wherein the compound of the formula I is contained in an amount of 40 to 90 % by weight, preferably 60 to 90 % by weight, the acrylic or methacrylic acid in an amount of 5 to 20 % by weight, preferably 5 to 30 % by weight, and the acrylic acid ester or methacrylic acid ester in an amount of 5 to 20 % by weight, preferably 5 to 30 % by weight, relative to the copolymer.

11. The use of a polymer according to Claim 6 as photographic recording material.

**Revendications**

1. Composés imidyliques ou mélanges de composés imidyliques répondant à la formule I

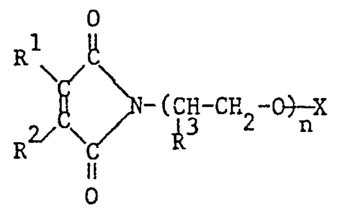

(I)

dans laquelle

$R^1$ et $R^2$ représentent chacun, indépendamment l'un de l'autre, un alkyle en $C_1$-$C_4$, ou

$R^1$ et $R^2$ forment ensemble un radical triméthylène ou tétraméthylène non substitué ou porteur d'un alkyle en $C_1$-$C_4$,

$R^3$ représente un atome d'hydrogène ou un alkyle en $C_1$-$C_4$,

n désigne un nombre de 2 à 30 et

X représente un atome d'hydrogène ou un radical $-CO-CR^4=CH_2$ dans lequel $R^4$ désigne un atome d'hydrogène ou un radical méthyle.

2. Composés selon la revendication 1, caractérisés en ce que $R^1$ et $R^2$ représentent chacun un radical méthyle ou forment ensemble un radical butylène-1,4 non substitué ou porteur d'un méthyle.

3. Composés selon la revendication 1, caractérisés en ce que $R^3$ représente un radical méthyle ou, mieux, un atome d'hydrogène.

4. Composés selon la revendication 1 dans lesquels n représente un nombre de 2 à 20, plus spécialement de 2 à 10 ou, mieux encore, de 2 à 6.

5. Composés selon la revendication 1 qui répondent à la formule suivante

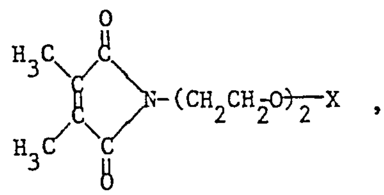

dans laquelle X a la signification donnée à la revendication 1.

6. Polymères réticulables sensibles à la lumière qui dérivent de composés de formule I selon la revendication 1 et copolymères avec des comonomères éthyléniques.

7. Polymères selon la revendication 6, caractérisés en ce qu'ils ont un poids moléculaire compris entre 1 000 et 1 000 000.

8. Polymères selon la revendication 6, caractérisés en ce que le comonomère insaturé présent dans le copolymère est un monomère vinylique.

9. Polymères selon la revendication 8, caractérisés en ce que le monomère vinylique est l'acide acrylique ou l'acide méthacrylique et/ou un ester de l'acide acrylique ou un ester de l'acide méthacrylique.

10. Polymères selon la revendication 9, caractérisés en ce que le composé de formule I y est contenu en une quantité de 40 à 90 % en poids, de préférence de 60 à 90 % en poids, l'acide acrylique ou méthacrylique en une quantité de 5 à 20 % en poids, de préférence de 5 à 30 % en poids, et l'ester acrylique

ou méthacrylique en une quantité de 5 à 20 % en poids, de préférence de 5 à 30 % en poids, par rapport au copolymère.

11. Application de polymères selon la revendication 6 comme matières d'enregistrement photographique.